# EUROPEAN PATENT APPLICATION

(11) **EP 4 115 950 A1**
(43) Date of publication of application: **11.01.2023**
(21) Application number: 21763653.9
(22) Date of filing: 01.03.2021
(51) Int. Cl.: A61P 1/00, A61P 1/10, A61P 37/04, A23L 33/125, A61K 31/724

(54) **INTESTINAL TRACT FUNCTION IMPROVING AGENT**

(30) Priority: 02.03.2020 JP 2020035185
(71) Applicant: Cyclochem Bio Co., Ltd., Kobe-shi, Hyogo 650-0047 (JP)
(72) Inventor: TERAO Keiji, Kobe-shi, Hyogo 650-0047 (JP); CHIKAMOTO Keita, Kobe-shi, Hyogo 650-0047 (JP); FURUNE Takahiro, Kobe-shi, Hyogo 650-0047 (JP); NANRI Ayumi, Yokohama-shi, Kanagawa 2310028 (JP); YOSHIKAWA Yutaka, Kobe-shi, Hyogo 650-0046 (JP)
(74) Representative: Forstmeyer, Dietmar
(86) International application number: PCT/JP2021/007772
(87) International publication number: WO 2021/177248

(57) **Abstract**

An agent which can play a comparable or superior role to LS in improvement of intestinal tract function, enhancement of immune function, suppression of production of a putrefactive product, improvement of bowel movement or the like and an agent which can play a role in protection of the intestinal tract from a food emulsifier, a lipid peroxide or the like are provided. An intestinal tract function-improving agent, an immunoenhancing agent, a putrefactive product production-suppressing agent, a bowel movement-improving agent and an intestinal tract protection agent each containing a cyclodextrin as an active ingredient are provided. they can play a comparable or superior role to LS in improvement of intestinal tract function, enhancement of immune function, suppression of production of a putrefactive products, improvement of bowel movement or the like and the agent which can play a role in protection of the intestinal tract from a food emulsifier, a lipid peroxide or the like. Using the agents as an active ingredient, an anti-inflammatory agent, an immunostimulant, a body odor-improving agent, a stool odor-improving agent, a laxative, an intestinal tract protection agent and the like can also be provided.

## Description

### TECHNICAL FIELD

The present invention relates to an intestinal tract function-improving agent containing a cyclodextrin (sometimes simply referred to as CD below) as an active ingredient. Furthermore, the invention relates to an immunoenhancing agent, a putrefactive product production-suppressing agent, a bowel movement-improving agent and an intestinal tract protection agent each containing a CD as an active ingredient.

### BACKGROUND ART

Human intestinal bacteria are known to decompose dietary fibers and indigestible oligosaccharides in the intestines and produce short-chain fatty acids (sometimes simply referred to as SCFAs below) which exert various health-promoting effects on the host.

Lactosucrose (LS), which is an indigestible oligosaccharide, is known to be useful for increasing the production amount of SCFAs. Intake of LS increases bifidobacteria and SCFAs in the intestines, and can have various effects such as promotion of calcium absorption into the body, regulation of immune function and suppression of production of an intestinal putrefactive product (for example, see Non-Patent Document 1). Thus, techniques using LS as an active ingredient of a drink composition for suppressing production of an intestinal putrefactive product or an anti-constipation composition or the like have been disclosed (for example, see Patent Documents 1 and 2).

The present inventors have focused on a CD, which is an indigestible oligosaccharide, as a new material which would replace LS. A CD is a substance having the inclusion complexation property and is used as encapsulating agents for various components of foods, drugs and the like.

For example, Patent Document 3 cites a CD as one of complexing agents for preparing a pharmaceutical composition which can be used for treating intestine-related disorders such as irritable bowel syndrome. Moreover, Patent Document 4 discloses use of a composition containing a *Quillaja saponaria-derived* saponin adjuvant and β-CD for the manufacture of a medicine for increasing or promoting immune response. Furthermore, Patent Document 5 discloses α-CD, β-CD, γ-CD and the like as saccharides in an anti-inflammatory agent that contains an inositol derivative in which a saccharide is bound to inositol as an active ingredient and that suppresses UV-caused inflammation.

As shown in the related art, however, the effects of a CD itself on intestinal tract function have not even been examined. Therefore, the inventors have attempted to provide an agent which can play a comparable or superior role to LS in improvement of intestinal tract function, enhancement of immune function, suppression of production of a putrefactive product, improvement of bowel movement or the like using a CD.

Moreover, it has been recently reported that emulsifiers contained in various foods cause toxicity to the cells of the intestinal tract, decrease the barrier function of the intestinal tract and increase membrane permeability of egg allergens or the like. Furthermore, it has also been reported that food-derived lipid peroxides induce oxidative stress, inflammation or the like in intestinal tract cells and cause cell death (see Non-Patent Documents 2 and 3).

Therefore, the inventors have also attempted to provide an agent which can play a role in protection of the intestinal tract from such a food emulsifier, a lipid peroxide or the like.

### CITATION LIST

### PATENT LITERATURE

Patent Document 1: Patent No. 2789069
Patent Document 2: Patent No. 4431316
Patent Document 3: JP-A-2019-116496
Patent Document 4: JP-A-2009-197014
Patent Document 5: JP-A-2019-112354

### NON PATENT LITERATURE

Non-Patent Document 1: Eriko KISHINO, et.al., Bioscience, Biotechnology, and Biochemistry, 2006, vol.70, no.6, p.1485-1488
Non-Patent Document 2: LorandKiss, et. al., J. Pharm. Sci., 2014, vol.103, p.3107-3119
Non-Patent Document 3: Subhashinee S K Wijeratne, et. al., J. Agric. Food Chem. 2006 vol.54, p.4476-4481

### SUMMARY OF INVENTION

### TECHNICAL PROBLEM

An object of the invention is to provide an agent which can play a comparable or superior role to LS in improvement of intestinal tract function, enhancement of immune function, suppression of production of a putrefactive product, improvement of bowel movement or the like. Another object of the invention is to provide an agent which can play a role in protection of the intestinal tract from a food emulsifier, a lipid peroxide or the like.

### SOLUTION TO PROBLEM

As a result of extensive investigation to achieve the objects, the inventors have found that a CD can play a comparable or superior role to LS in improvement of intestinal tract function, enhancement of immune function, suppression of production of a putrefactive product, improvement of bowel movement or the like and that a CD can play a role in protection of the intestinal tract from a food emulsifier, a lipid peroxide or the like. The invention has been thus completed.

That is, the invention relates to an intestinal tract function-improving agent and the like shown in (1) to (5) below.
(1) An intestinal tract function-improving agent containing a CD as an active ingredient.
(2) An immunoenhancing agent containing a CD as an active ingredient.
(3) A putrefactive product production-suppressing agent containing a CD as an active ingredient.
(4) A bowel movement-improving agent containing a CD as an active ingredient.
(5) An intestinal tract protection agent containing a CD as an active ingredient.

### ADVANTAGEOUS EFFECTS OF INVENTION

According to the invention, an agent which can play a comparable or superior role to LS in improvement of intestinal tract function, enhancement of immune function, suppression of production of a putrefactive product, improvement of bowel movement or the like and an agent which can play a role in protection of the intestinal tract from a food emulsifier, a lipid peroxide or the like can be provided.

The agents obtained by the invention can be used directly as a food, a health food, a quasi-drug, a pharmaceutical or the like or used as an active ingredient of an anti-inflammatory agent, an immunostimulant, a body odor-improving agent, a stool odor-improving agent, a laxative, an intestinal tract protection agent or the like, and the agents can be thus used for various applications.

### BRIEF DESCRIPTION OF DRAWINGS

[Fig. 1] A figure showing the effects on improvement of intestinal tract function (Test Example 1).
[Fig. 2] A figure showing the effects on enhancement of immune function (Test Example 1).
[Fig. 3] A figure showing the effects on suppression of production of putrefactive products (Test Example 1).
[Fig. 4] A figure showing the effects on improvement of bowel movement (A. wet weight and B. dry weight) (Test Example 1).
[Fig. 5] A figure showing the effects on cytotoxicity of food emulsifiers (A. HLB16P, B. HLB11S, C. HLB16S and D: monolaurin) (Test Example 2).
[Fig. 6] A figure showing comparison of the effects with other water-soluble dietary fibers or oligosaccharides (Test Example 2).
[Fig. 7] A: A figure showing the effects on decrease in the transepithelial electrical resistance value of a food emulsifier (Test Example 2). B: A figure comparing the effects with other water-soluble dietary fibers or oligosaccharide (Test Example 2).
[Fig. 8] A: A figure showing the effects on cytotoxicity of a lipid peroxide (Test Example 2). B: A figure showing comparison of the effects with other water-soluble dietary fibers or oligosaccharides (Test Example 2).

### DESCRIPTION OF EMBODIMENTS

The "intestinal tract function-improving agent", the "immunoenhancing agent", the "putrefactive product production-suppressing agent", the "bowel movement-improving agent" and the "intestinal tract protection agent" of the invention each mean an agent which exhibits an effect on improvement of intestinal tract function, enhancement of immune function, suppression of production of a putrefactive product, improvement of bowel movement or intestinal tract protection in an animal which has taken the agent when taken by an animal such as a human, a mouse, a rat, a rabbit, a dog, a cat, a horse, a sheep, a monkey and a cow. The "intestinal tract function-improving agent", the "immunoenhancing agent", the "putrefactive product production-suppressing agent", the "bowel movement-improving agent" and the "intestinal tract protection agent" of the invention are each an agent containing a CD as an active ingredient. The agents may be a CD itself or may be an agent further containing, in addition to the CD, another component such as a component which can support improvement of intestinal tract function or the like of LS or the like and a component necessary for keeping the dosage form.

The CD which can be used as an active ingredient of the "intestinal tract function-improving agent", the "immunoenhancing agent", the "putrefactive product production-suppressing agent", the "bowel movement-improving agent" and the "intestinal tract protection agent" of the invention is a CD which an animal can take safely and may be a commercial CD or a self-made CD. Such CDs are α-CD, β-CD, γ-CD, maltosyl-α-CD, maltosyl-β-CD, maltosyl-γ-CD and the like. One kind of the CDs may be used as an active ingredient, or two or more kinds thereof may be used in combination.

Examples of commercial α-CD include pure fiber (COSANA Corporation), nansyokasei α-origoto (COSANA Corporation) and the like.

An effect which is improved by the "intestinal tract function-improving agent" of the invention is an increase in the production amount of mucin. Due to the function, the "intestinal tract function-improving agent" of the invention can be used for protection of the mucosa of the intestinal tract, promotion of colonization of a useful microorganism such as lactic acid bacteria in the intestinal tract or the like. Moreover, an effect which is enhanced by the "immunoenhancing agent" of the invention is an increase in the production amount of IgA, and due to the function, immune function of the intestinal tract or the like can be enhanced.

The putrefactive products whose production is suppressed by the "putrefactive product production-suppressing agent" of the invention are indole, p-cresol, phenol and the like, and body odor or stool odor can be improved through a decrease in the production amount thereof. In addition, the "bowel movement-improving agent" of the invention can exhibit an effect of increasing feces amount or the like and can be used for improvement of constipation or the like.

Furthermore, the "intestinal tract protection agent" of the invention can suppress or improve the cytotoxicity in intestinal tract cells, a decrease in transepithelial electrical resistance or the like due to a food emulsifier, a lipid peroxide or the like and can protect the intestinal tract.

The "intestinal tract function-improving agent", the "immunoenhancing agent", the "putrefactive product production-suppressing agent", the "bowel movement-improving agent" and the "intestinal tract protection agent" of the invention may be administered in an amount which exhibits an effect in an animal which takes the agent. For example, when the animal which takes such an agent is a human, the agent is preferably administered in such a manner that the amount of the CD which is contained in the agent and is taken becomes around 0.001 g to 10 g/day.

### EXAMPLES

The samples used in the Examples, the Test Examples and the like of the invention are shown below.

### <Samples>

### 1. α-CD (CAVAMAX W6 Food, CYCLOCHEM BIO CO., LTD.)

### 2. LS (Origo no Okage LS-90P, ENSUIKO Sugar Refining Co., Ltd.)

### 3. Caco-2 Cells

Caco-2 cells (human colon cancer-derived cell line) (KAC Co., Ltd.) which were cultured in a medium obtained by mixing 10% FBS (SIGMA) and 1% Antibiotic-Antimycotic Mixed Stock Solution (100x) (Nacalai Tesque, Inc.) in Dulbecco's Modified Eagle's Medium (SIGMA) were used.

### 4. Food Emulsifiers

### 1) Sucrose fatty acid esters (SEs) (product name: Ryoto (trademark) Sugar Ester) (Mitsubishi-Chemical Foods Corporation)

The SEs in (1) to (3) below were used. HLB is a value indicating the degree of affinity of a surfactant to water and oil. A smaller value indicates higher lipophilic property, and a larger value indicates higher hydrophilic property.

### (1) HLB16P (product name: P-1670)

A sucrose ester modified with palmitic acid which is generally used for frozen bread dough and the like.

### (2) HLB11S (product name: S-1170)

A sucrose ester modified with stearic acid which is generally used for wheat flour products, fish paste products and the like.

### (3) HLB16S (product name: S-1670)

A sucrose ester modified with stearic acid which is generally used for wheat flour products, fish paste products, frozen bread dough and the like.

### 2) Glycerol fatty acid ester

Monolaurin (monoglycerol fatty acid ester, Tokyo Chemical Industry Co., Ltd.)

### 5. Lipid Peroxide

Oxidized oleic acid prepared by irradiating oleic acid (FUJIFILM Wako Pure Chemical Corporation) with ultraviolet rays (maximum wavelength of 253.7 nm, UV output of 1 W) at room temperature for 40 hours using TOP (registered trademark) UV box (Sogo Laboratory Glass Works Co., Ltd) was used.

### 6. Water-Soluble Dietary Fibers or Oligosaccharides

1) Inulin (IN) (inulin, FUJIFILM Wako Pure Chemical Corporation)
2) Hydrolyzed guar gum (PHGG) (Sunfiber, Taiyo-labo, TL1)
3) Lactosucrose (LS) (*Origo no Okage* LS-90P, ENSUIKO Sugar Refining Co., Ltd.)
4) Indigestible Dextrin (ID) (Fibersol-2, Matsutani Chemical Industry Co., Ltd.)
5) Polydextrose (PDX) (STA-LITE^{®} III, Koyo Mercantile Co., Ltd.)
6) Isomaltodextrin (IMD) (Fibryxa TM, Hayashibara Co., Ltd.)
7) Galactooligosaccharide (GOS) (galactooligosaccharide, FUJIFILM Wako Pure Chemical Corporation)
8) Fructooligosaccharide (FOS) (fructooligosaccharide, FUJIFILM Wako Pure Chemical Corporation)
9) Trehalose (Tre) (trehalose, Marugo Corporation)
10) Mannitol (MNT) (D(-)-mannitol, FUJIFILM Wako Pure Chemical Corporation)

### [Example 1]

### Production Method of Intestinal Tract Function-Improving Agent

α-CD was directly used as an intestinal tract function-improving agent.

### [Example 2]

### Production Method of Immunoenhancing Agent

α-CD was directly used as an immunoenhancing agent.

### [Example 3]

### Production Method of Putrefactive product Production-Suppressing Agent

α-CD was directly used as a putrefactive product production-suppressing agent.

### [Example 4]

### Production Method of Bowel Movement-Improving Agent

α-CD was directly used as a bowel movement-improving agent.

### [Example 5]

### Production Method of Intestinal Tract Protection Agent

α-CD was directly used as an intestinal tract protection agent.

### [Test Example 1]

### Examination of Effects

### 1. Test Method

Fifteen 3-week-old SD rats (Japan SLC, Inc.) were acclimatized to the facility for one week and then divided into three groups of NF group (No Fiber diet), α-CD group (5.5% α-CD-containing NF diet) and LS group (5.5% LS-containing NF diet), each including five rats, and each rat was kept in one cage. In this test, the experiment was conducted in accordance with the Guidelines for Animal Experimentation of Kobe Women's University (Kobe, Japan), and approved by the animal ethics committee of the Graduate School of Science of Health and Nutrition at Kobe Women's University. The breeding environment was at 23±3°C with a humidity of 60±10%, and cycles of 12-hour light/12-hour dark were provided. The rats were allowed to freely take diets and water and bread for eight weeks with experimental diets.

The blending ratio of experimental diet for each group is shown in Table 1. The diets were prepared in such a manner that the α-CD or LS content of dry experimental diet became 5.5%. After mixing each powder well, 100 ml of water was added, and thus mixed material was used as an experimental diet.

The rats of each group were fasted overnight eight weeks after starting breeding and then dissected under isoflurane anesthesia, and the cecum were taken out.

**[Table 1]**

| Blending Ratio of Experimental Diet | | | |
|---|---|---|---|
| | NF Group | α-CD Group | LS Group |
| Casein | 180 | 180 | 180 |
| Corn Oil | 50 | 50 | 50 |
| Mineral Mix* | 35 | 35 | 35 |
| Vitamin Mix** | 10 | 10 | 10 |
| L-cystine | 2 | 2 | 2 |
| t-Butylhydroquinone | 0.008 | 0.008 | 0.008 |
| Choline Bitartrate | 2.5 | 2.5 | 2.5 |
| Sucrose | 520 | 520 | 520 |
| Glucose | 200 | 139 | 142 |
| α-CD | - | 61 | - |
| LS | - | - | 58 |
| Total Amount (g) | 1000 | 1000 | 1000 |

| | | | |
|---|---|---|---|
| Mineral Mix* : CLEA Japan, Inc. Vitamin Mix** : CLEA Japan, Inc. | | | |

### 2. Analysis of Cecal Microflora and Short-Chain Fatty Acid Amounts

A cut was made in the cecum taken out in 1 above, and the contents were squeezed out. The weights of the cecal contents were measured. Immediately after the weight measurement, the contents were frozen with liquid nitrogen and stored at -80°C.

The cecal contents which were thawed for the microflora analysis were mixed and homogenized, and the analysis of the microflora by the T-RFLP method, measurement of the SCFAs amounts and the pH analysis were entrusted (TechnoSuruga Laboratory Co., Ltd.). The total SCFAs amounts were calculated by adding up the amounts of lactic acid, succinic acid, acetic acid, propionic acid and butyric acid.

As a result, in the analysis of the cecal microflora by the T-RFLP method, the proportion of the Lactobacillales increased in the α-CD group as compared to the NF group or the LS group. In the analysis of the SCFAs amounts, it was shown that the amounts of lactic acid, succinic acid, acetic acid, propionic acid and n-butyric acid and the total SCFAs amount increased significantly in the α-CD group as compared to the NF group, and it was confirmed that the increases in the amounts of lactic acid, succinic acid, acetic acid, propionic acid and n-butyric acid and the total SCFAs amount were remarkable as compared to those of the LS group. It was shown that the cecal pH decreased significantly in the α-CD group as compared to the NF group, and it was confirmed that the decrease in the cecal pH was remarkable as compared to that of the LS group. The results suggest the possibility that the intake of α-CD increases the SCFAs amounts more than LS under normal diet.

### 3. Effects on Intestinal Tract Function

### 1) Mucin Measurement

The mucin amounts of the cecal contents were measured using Fecal mucin Assay Kit (Cosmo Bio Co., Ltd.), statistically analyzed by the Dunnett's test and the Student's t-test using Pharmaco Analyst I (Ver. 13.1.1; Three S Japan) and shown with the average values ± standard errors.

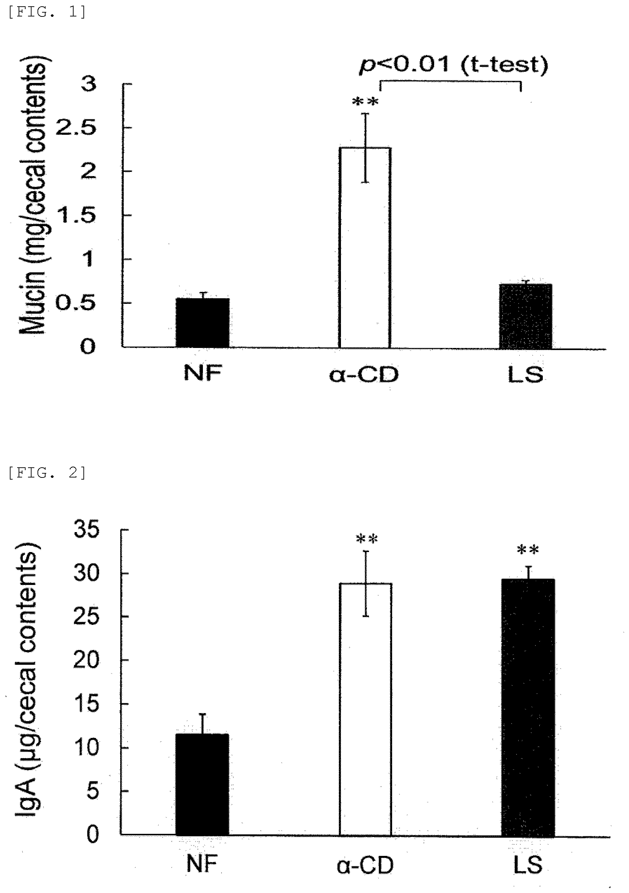

As shown in Fig. 1, the results showed that the mucin amount increased significantly in the group taking α-CD-containing experimental diet (the α-CD group) as compared to the group taking α-CD- and LS-free experimental diet (the NF group) (p<0.01). Moreover, as a result of statistical analysis between the two groups, the α-CD group and the group taking LS-containing experimental diet (the LS group), by the Student's t-test, it was confirmed that the mucin amount increased significantly in the α-CD group as compared to the LS group (p<0.01).

### 2) IgA Measurement

One hundred milligrams of cecal contents were measured and stirred after adding 1 ml of Elix water. After stirring, the resultant was centrifuged under the conditions of 10000 rpm for 30 min at 4°C, and the supernatant was used as a sample for IgA measurement. IgA amount of each sample was measured using Rat IgA ELISA Kit (Bethyl Laboratories, Inc.). Statistical analysis was conducted in the same manner as in 1) above.

As a result, as shown in Fig. 2, it was confirmed that the IgA amount increased significantly in the α-CD group, like in the LS group, as compared to the group taking α-CD- and LS-free experimental diet (the NF group) (p<0.01). Moreover, as a result of statistical analysis between the two groups, the α-CD group and the group taking LS-containing experimental diet (the LS group), by the Student's t-test, it was confirmed that α-CD exhibited IgA-increasing effect that is comparable to that of LS.

The results of 1) and 2) above showed that the intake of the CD increases the production amount of mucin in the intestinal tract and can further protect the mucosa of the intestinal tract and promote colonization of a useful microorganism such as lactic acid bacteria in the intestinal tract or the like more than the intake of LS. Moreover, because IgA is produced in an amount comparable to that of the intake of LS and can improve the immune function of the intestinal tract, it was shown that the intake of the CD can improve intestinal tract function and can be used for suppression of onset of inflammation such as enteritis, relief of inflammation, protection of the intestinal tract or the like.

### 4. Putrefactive product Measurement

Analysis of the amounts of putrefactive products (indole, p-cresol and phenol) in cecal contents was entrusted (TechnoSuruga Laboratory Co., Ltd.).

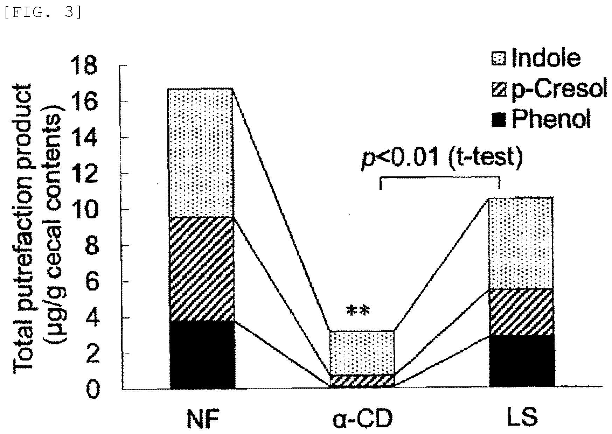

As shown in Fig. 3, the results showed that the amounts of the putrefactive products decreased significantly in the group taking α-CD-containing experimental diet (the α-CD group) as compared to the group taking α-CD- and LS-free experimental diet (the NF group) (p<0.01). Moreover, as a result of statistical analysis between the two groups, the α-CD group and the group taking LS-containing experimental diet (the LS group), by the Student's t-test, it was confirmed that the production of the putrefactive products was suppressed significantly in the α-CD group as compared to the LS group (p<0.01). Accordingly, the results showed that the intake of the CD can suppress the production of putrefactive products and can be used for suppression of body odor or feces odor or the like even more than the intake of LS.

### 5. Feces Amount Measurement

A drainboard was placed on the floor of each breeding cage 44 days after starting the test, and the feces that fell under the drainboard were collected every day for three days in succession after the 45th day. The weight (wet weight) of the collected feces (for the three days) was measured, and then after drying under reduced pressure, the feces weight after drying (dry weight) was measured.

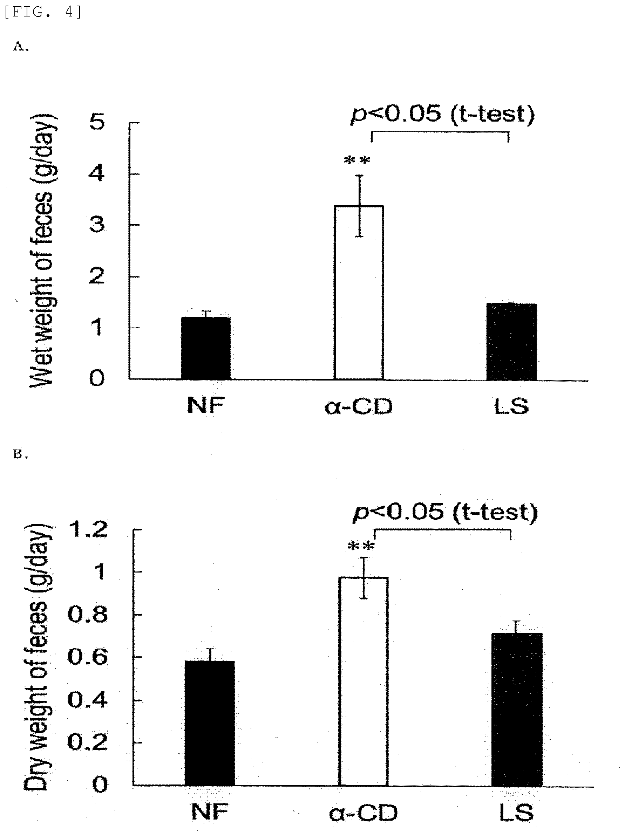

As shown in Figs. 4A and B, the results showed that the feces amounts, both the wet weight and the dry weight, increased significantly in the group taking α-CD-containing experimental diet (the α-CD group) as compared to the group taking α-CD- and LS-free experimental diet (the NF group) (p<0.01). Moreover, as a result of statistical analysis between the two groups, the α-CD group and the group taking LS-containing experimental diet (the LS group), by the Student's t-test, it was confirmed that the feces amounts, both the wet weight and the dry weight, increased significantly in the α-CD group as compared to the LS group (p<0.05). Accordingly, the results showed that the intake of the CD improves bowel movement and can be used for effect of improving bowel movement or the like even more than the intake of LS.

### [Test Example 2]

### Examination of Intestinal Tract Protection Effects

### 1. Intestinal Tract Protection from Food Emulsifiers

### [Cytotoxicity]

### 1) Effects of Emulsifiers

Caco-2 cells were inoculated to a 96-well plate at 10000 cells/well and cultured for three days to confluency. The Caco-2 cells were washed with PBS and then treated with a food emulsifier SE (250 µg/mL HLB16P, 1000 µg/mL HLB11S or 500 µg/mL HLB11S) or monolaurin (500 µg/mL) and α-CD for an hour, and the LDH activities of each were measured. The treatment amounts of α-CD were 1/10, 1, 1.5, 2, 5 and 10 times the weight ratios for each SE. For monolaurin, the treatment amounts of α-CD were 1/10, 1, 1.5, 2, 5, 10 and 15 times the weight ratios.

For comparison, the LDH activities of Caco-2 cells which were treated with the SE (HLB16P (0-1000 µg/mL), HLB11S or HLB16S (each 0-2000 µg/mL)) or monolaurin (0-2500 µg/mL) alone for an hour were also measured.

As a result, when each SE alone was contained, it was confirmed as follows: the cytotoxicity to the Caco-2 cells was observed with HLB16 at 250 µg/mL or more and was very high at 500 µg/mL or more; the cytotoxicity was observed with HLB11S at 62.5 µg/mL or more and was very high at 2000 µg/mL; and the cytotoxicity was observed with HLB16S at 250 µg/mL or more and was very high at 1000 µg/mL or more. When monolaurin alone was contained, it was confirmed that the cytotoxicity to the Caco-2 cells was observed at 156.25 µg/ml or more and was very high at 625 µg/mL or more.

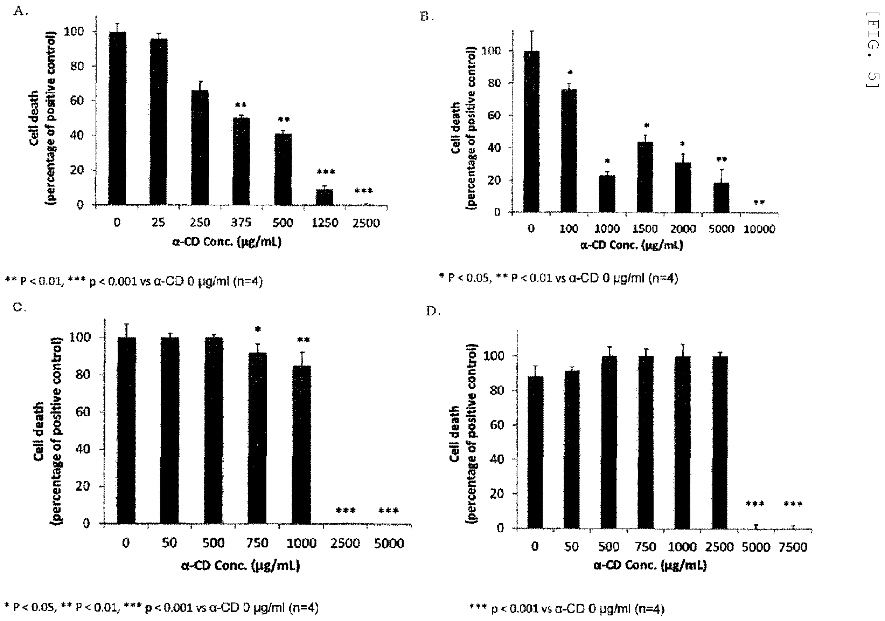

On the other hand, when each SE and α-CD were contained in combination, it was confirmed that the cytotoxicity of HLB16P (250 µg/mL) was reduced significantly with the addition of α-CD in 1.5 times the amount or more and that the cytotoxicity disappeared completely with the addition of 10 times the amount (Fig. 5A).

It was also confirmed that the cytotoxicity of HLB11S (1000 µg/mL) was reduced significantly with the addition of α-CD in 0.1 times the amount or more and that the cytotoxicity disappeared completely with the addition of 10 times the amount (Fig. 5B).

It was further confirmed that the cytotoxicity of HLB16S (500 µg/mL) was reduced significantly with the addition of α-CD in 1.5 times the amount or more and that the cytotoxicity disappeared completely with the addition of 5 times the amount (Fig. 5C).

It was also confirmed that the cytotoxicity of monolaurin (500 µg/mL) disappeared completely with the addition of α-CD in 10 times the amount or more (Fig. 5D).

### 2) Comparison with Water-Soluble Dietary Fibers or Oligosaccharides

Caco-2 cells which were cultured in a 96-well plate to confluency in the same manner as in 1) above were treated with 1000 µg/mL of HLB11S and 1000 µg/mL of each water-soluble dietary fiber or an oligosaccharide for an hour, and the LDH activities were measured.

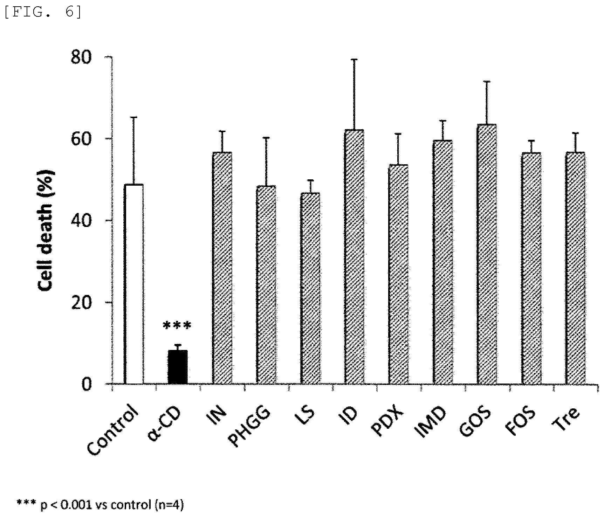

As a result, as shown in Fig. 6, it was confirmed that the cytotoxicity of the food emulsifier was not suppressed at all by the water-soluble dietary fibers or the oligosaccharides other than α-CD and that only α-CD exhibits such an effect.

### [Transepithelial electrical resistance]

Caco-2 cells were seeded to a Transwell insert at 0.4×10⁶ cells/mL using Corning (registered trademark) BioCoat (trademark) intestinal epithelial cells (Caco-2) differentiation environment kit (Corning International K.K.), cultured for one day in a normal medium and cultured for three days in a differentiation medium. The Caco-2 cells were washed with PBS, then treated with HLB16P (62.5 µg/mL) and α-CD (0-2000 µg/mL) and incubated for 120 minutes. During the incubation, the transepithelial electrical resistance values were measured every 20 minutes by Millicell ERS-2 resistance measurement system (Nihon Millipore K.K.).

In the same manner, the transepithelial electrical resistance values were measured after treatment with HLB16P (62.5 µg/mL) and α-CD (2000 µg/mL), each water-soluble dietary fiber or oligosaccharide (2000 µg/mL).

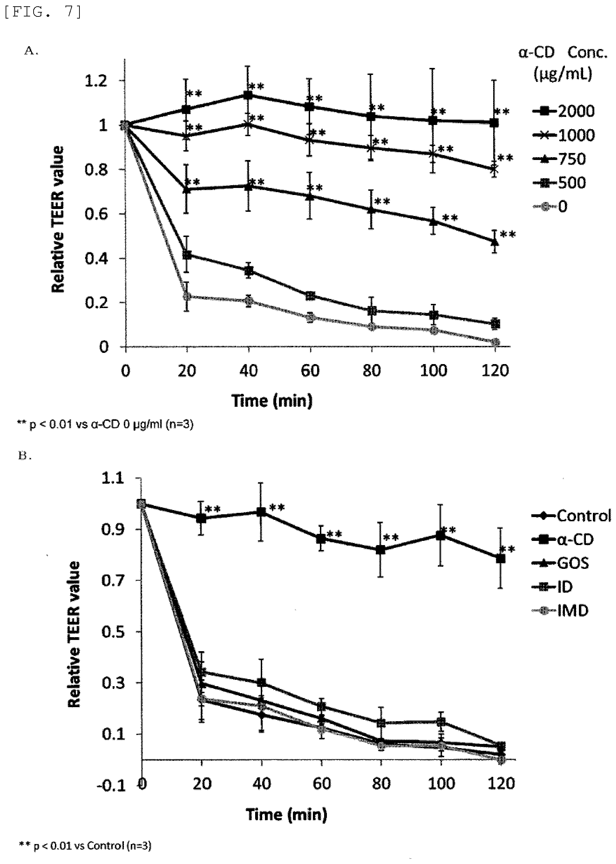

As a result, it was confirmed that the decrease in transepithelial electrical resistance value by the SE was suppressed by the addition of α-CD in a concentrationdependent manner (Fig. 7A). It was also confirmed that the decrease in transepithelial electrical resistance value by the SE was not suppressed at all by the other water-soluble dietary fibers or oligosaccharide (Fig. 7B).

### 2. Intestinal Tract Protection to Lipid Peroxide

### [Cytotoxicity]

Caco-2 cells which were cultured in a 96-well plate to confluency in the same manner as in 1, 1) above were washed with PBS and then treated for two hours with oxidized oleic acid (0-2 mM) which was diluted with an aqueous taurocholic acid solution (final concentration of 3.8 mM), and the cytotoxicity was measured with Cytotoxicity LDH Assay Kit-WST (Dojindo Laboratories).

Caco-2 cells were similarly treated for two hours with mixtures of oxidized oleic acid (0.5 mM) and α-CD (mole ratio of 1:1, 1:2, 1:4 or 1:6) which were stirred and left still for 20 minutes, and the cytotoxicity was measured. Moreover, the cytotoxicity was similarly measured also using mixtures of oxidized oleic acid (0.5 mM) and 2 mM α-CD or another water-soluble dietary fiber at the equivalent dose to that of α-CD which were stirred and left still for 20 minutes.

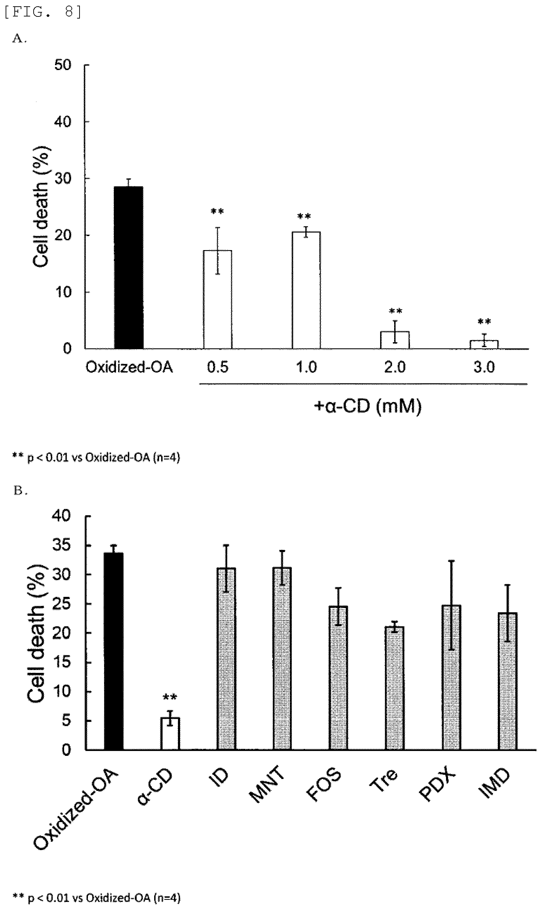

When the cells were treated with oxidized oleic acid alone, cytotoxicity which was dependent on the treatment concentration of oxidized oleic acid was observed. On the other hand, when the cells were treated with oxidized oleic acid and α-CD, it was confirmed that the cytotoxicity of oxidized oleic acid was suppressed dependently on the α-CD concentration (Fig. 8A).

It was also shown that the cytotoxicity of the lipid peroxide was not suppressed at all by the water-soluble dietary fibers or the oligosaccharides other than α-CD and that only α-CD has such an effect (Fig. 8B).

### 3. Conclusion

From 1 and 2 above, it was confirmed that α-CD has effects on suppression or complete loss of cytotoxicity of food emulsifiers or lipid peroxides to intestinal tract cells and on suppression of a decrease in transepithelial electrical resistance. Such effects of α-CD were not observed with other water-soluble dietary fibers and oligosaccharides and were shown to be specific to α-CD.

Accordingly, these results suggested that intake of the intestinal tract protection agent of the invention containing a CD as an active ingredient can protect the intestinal tract from food emulsifiers, which is contained in various foods and drinks, from lipid peroxides, which is generated through storage or the like, or from the like. Furthermore, they also suggested that intake or the like of the intestinal tract protection agent of the invention before meals or the like can relieve damage to the intestinal tract.

### INDUSTRIAL APPLICABILITY

According to the invention, an agent which can play a comparable or superior role to LS in improvement of intestinal tract function, enhancement of immune function, suppression of production of a putrefactive product, improvement of bowel movement or the like and an agent which can play a role in protection of the intestinal tract from a food emulsifier, a lipid peroxide or the like can be provided. The agents obtained by the invention can be used directly as a food, a health food, a quasi-drug, a pharmaceutical or the like or used as an active ingredient of an anti-inflammatory agent, an immunostimulant, a body odor-improving agent, a stool odor-improving agent, a laxative, an intestinal tract protection agent or the like, and the agents can be thus used for various applications.

## Claims

1. An intestinal tract function-improving agent containing a cyclodextrin as an active ingredient.

2. An immunoenhancing agent containing a cyclodextrin as an active ingredient.

3. A putrefactive product production-suppressing agent containing a cyclodextrin as an active ingredient.

4. A bowel movement-improving agent containing a cyclodextrin as an active ingredient.

5. An intestinal tract protection agent containing a cyclodextrin as an active ingredient.
